# EUROPÄISCHE PATENTSCHRIFT

(11) **EP 3 140 633 B1**
(45) Veröffentlichungstag und Bekanntmachung des Hinweises auf die Patenterteilung: **12.02.2020**
(21) Anmeldenummer: 15707679.5
(22) Anmeldetag: 05.03.2015
(51) Int. Cl.: G01N 21/3504, G01N 21/85

(54) **SPEKTROSKOPISCHE SENSORVORRICHTUNG UND VERFAHREN ZUM BETREIBEN EINER SPEKTROSKOPISCHEN SENSORVORRICHTUNG**
SPECTROSCOPIC SENSOR DEVICE AND METHOD FOR APPLYING A SPECTROSCOPIC SENSOR DEVICE
DISPOSITIF SPÉCTROSCOPIQUE À CAPTEURS ET PROCÉDÉ D'UTILISATION D'UN DISPOSITIF SPÉCTROSCOPIQUE À CAPTEURS

(30) Priorität: 06.05.2014 DE 102014208382
(43) Veröffentlichungstag der Anmeldung: 15.03.2017
(73) Patentinhaber: Robert Bosch GmbH, 70442 Stuttgart (DE)
(72) Erfinder: KOOP, Paul, 70567 Stuttgart (DE)
(86) Internationale Anmeldenummer: PCT/EP2015/054623
(87) Internationale Veröffentlichungsnummer: WO 2015/169472

(56) Entgegenhaltungen:
- EP-A1- 1 070 956
- WO-A1-01/69208
- DE-A1-102006 049 260
- DE-A1-102009 027 134
- US-B1- 8 559 721

## Beschreibung

Die vorliegende Erfindung betrifft eine spektroskopische Sensorvorrichtung und ein Verfahren zum Betreiben einer spektroskopischen Sensorvorrichtung.

### Stand der Technik

Spektroskopische Sensoren dienen dazu, in zu untersuchenden fluiden Medien, insbesondere in Gasen oder Flüssigkeiten, Konzentrationen bestimmter Substanzen durch die Absorption von IR-Strahlung in relevanten Wellenlängenbereichen zu ermitteln.

Die bekannten spektroskopischen Sensoren nutzen in der Regel einen breitbandigen Infrarotstrahler und einen Infrarotdetektor mit einem oder mehreren Detektorkanälen. Üblicherweise wird für jede Gas- bzw. Flüssigkeitsspezies ein Detektorkanal als Messkanal verwendet, wobei ein weiterer Detektorkanal als Referenzkanal verwendet wird.

Insbesondere sind zweikanalige Detektoren bekannt, um eine bestimmte Substanz zu detektieren. Bei einem derartigen zweikanaligen Detektor wird ein erster Detektorkanal für die Detektion der Strahlung in einem relevanten Wellenlängenbereich, in dem eine Absorptionsbande der zu ermittelnden bzw. zu untersuchenden Substanz liegt, und der zweite Detektorkanal als Referenzkanal zur Detektion in einem breiteren Wellenlängenbereich verwendet. Die Selektion der Wellenlängenbereiche erfolgt im Allgemeinen durch Verwendung optischer Filter, die für bestimmte Wellenlängen bzw. Wellenlängenbereiche transparent sind.

Die DE 10 2009 027 136 A1 beschreibt einen spektroskopischen Sensor, der eine IR-Strahlungsquelle zum Aussenden von IR-Strahlung, eine Absorptionsstrecke zur Aufnahme eines Gases oder einer Flüssigkeit, mindestens ein optisches Filter zur wellenlängenselektiven Transmission der durch die Absorptionsstrecke gelangten IR-Strahlung und mindestens einen Detektor zur Aufnahme der durch das optische Filter gelangenden IR-Strahlung und Ausgabe eines Messsignals aufweist. Die IR-Strahlungsquelle und der Detektor sind an einem Schaltungsträger befestigt und kontaktiert, und die Absorptionsstrecke ist als Innenraum einer Reflektoreinrichtung ausgebildet, die an dem Schaltungsträger befestigt ist und eine reflektierende Innenfläche aufweist, sodass eine direkte Strahlungsübertragung von der IR-Strahlungsquelle zu dem Detektor ohne Reflektion an der Reflektoreinrichtung verhindert ist.

Die DE 10 2009 027 134 A1 beschreibt einen spektroskopischen Sensor, welcher eine Absorptionsstrecke zum Aufnehmen einer zu untersuchenden Substanz, eine IR-Strahlungsquelle zum Aussenden von IR-Strahlung durch die Absorptionsstrecke, eine Detektoreinrichtung, die mindestens ein erstes und ein zweites Detektorelement zum Detektieren der durch die Absorptionsstrecke gelangten IR-Strahlung und zum Ausgeben von Messsignalen aufweist. Eine Auswerteeinrichtung ist vorgesehen, die die Messsignale der mindestens zwei Detektorelemente aufnimmt und einen Gehalt an einem ersten Medium ermittelt, wobei das erste Detektorelement IR-Strahlung in einem ersten Wellenlängenbereich detektiert und ein erstes Messsignal ausgibt, das zweite Detektorelement IR-Strahlung in einem von dem ersten Wellenlängenbereich verschiedenen zweiten Wellenlängenbereich detektiert und ein zweites Messsignal ausgibt. In dem ersten Wellenlängenbereich liegt ein Absorptionsband des ersten Mediums und eines zweiten Mediums. In dem zweiten Wellenlängenbereich liegt ein Absorptionsband entweder des ersten Mediums oder des zweiten Mediums, wobei die Auswerteeinrichtung das zweite Messsignal zur Korrektur des ersten Messsignals aufnimmt.

Ein weiterer Detektor nach dem Stand der Technik ist aus der US-A-8559721 bekannt.

### Offenbarung der Erfindung

Die vorliegende Erfindung schafft eine spektroskopische Sensorvorrichtung mit den Merkmalen des unabhängigen Anspruchs 1 sowie ein Verfahren zu Betreiben einer spektroskopische Sensorvorrichtung mit den Merkmalen des unabhängigen Anspruchs 9.

Bevorzugte Weiterbildungen sind Gegenstand der Unteransprüche.

### Vorteile der Erfindung

Die der vorliegenden Erfindung zugrundeliegende Idee ist es, ein Infrarot-Sensorarray vorzusehen, welches eine Vielzahl von einzeln auswertbaren Pixeln aufweist, welche ausgebildet sind, die von der Infrarot-Strahlungsquelle durch das fluide Medium propagierte Infrarotstrahlung als einzelne Pixelmesssignale zu detektieren, wobei eine Auswerteeinrichtung vorgesehen ist, welche ausgebildet ist, eine vorgebbare Mehrzahl von Pixelmessignalen der Pixel zusammenzufassen, und über einen einzelnen Messkanal auszugeben.

Durch die Verwendung eines Infrarot-Sensorarrays mit mehreren auswählbaren bzw. ansteuerbaren Pixeln für einen einzelnen Messkanal wird eine einfache Möglichkeit zur internen Selbstanalyse für sicherheitsrelevante Anwendungen geschaffen.

Insbesondere lässt sich die Empfindlichkeit durch eine lokale Verschmutzung im optischen Pfad der Messtrecke reduzieren bzw. eliminieren. Des Weiteren kann die Empfindlichkeit gegenüber mechanischen oder sonstigen Fertigungstoleranzen bei der Produktion der Sensorvorrichtung reduzieren. Überdies werden die Messempfindlichkeit und das Signal-Rausch-Verhältnis der Sensorvorrichtung durch eine gleichzeitige Verwendung von mehreren einzeln auswertbaren Pixeln erhöht. Insbesondere kann die Flexibilität für unterschiedliche Gase verbessert werden. Auch können die Korrekturmöglichkeiten der Abweichungen durch Umwelteinflüsse/Alterung deutlich verbessert werden.

Als Infrarot-Strahlungsquelle kann beispielsweise eine breitbandige Strahlungsquelle, wie z. B. eine Glühbirne oder einen Heizdraht, oder eine schmalbandige Strahlungsquelle, wie z. B. eine Leuchtdiode oder einen Laser, verwendet werden. Die Absorptionsstrecke kann mit ein Einlass- und Auslassöffnungen versehen sein, sodass das zu untersuchende Gas oder Fluid in die Absorptionsstrecke und aus der Absorptionsstrecke fließen kann.

Gemäß der vorliegenden Erfindung sind die Pixel ein oder mehrerer Pixelbereiche zumindest teilweise mit einem jeweiligen optischen Filter zur wellenlängenselektiven Transmission der Infrarotstrahlung versehen .

Die Auswerteeinrichtung ist ausgebildet, die Position des oder der optischen Filter zu bestimmen. Der oder die Filter können beispielsweise durch produktionsbedingte Fehler nicht exakt auf der vorherbestimmten Position auf den Pixeln des Sensorarrays angeordnet sein, wodurch bestimmte Pixel nicht vollständig von einem Filter abgedeckt sind. Durch eine Messung der einzelnen Pixelmesssignale der einzelnen Pixel kann eine Positionsbestimmung der Filter auf dem Infrarot-Sensorarray durch die Auswerteeinrichtung vorgenommen werden. Die Auswerteeinrichtung ist dann beispielsweise dazu ausgebildet, lediglich Pixelmesssignale von Pixeln beim Zusammenfassen zu berücksichtigen, welche vollständig von einem Filter bedeckt sind. Pixel und Pixelbereiche, welche nicht von einem Filter abgedeckt sind, werden bei einer Messung nicht von der Auswerteeinrichtung berücksichtigt. Auf diese Weise wird die Messgenauigkeit des Sensors weiter erhöht. Die exakte Position der Filter kann in der Auswerteinrichtung in einem Speicher abgespeichert werden.

Gemäß einer Ausführungsform der vorliegenden Erfindung sind zumindest zwei Pixel des Infrarot-Sensorarrays ausgebildet, ein erstes Gas zu detektieren, und mit einem ersten optischen Filter zur wellenlängenselektiven Transmission der Infrarotstrahlung versehen. Der Filter kann beispielsweise auf der Chip-Kappe des Sensorarray geklebt sein, und/oder durch eine mechanische Verbindung, z. B. Schrauben, mit der Chip-Kappe des Sensorarrays verbunden sein. Beispielsweise weist das Sensorarray zwei Zeilen Pixel, und zwei Spalten Pixel auf, wobei zumindest zwei der vier Pixel mit dem ersten Filter zur wellenlängenselektiven Transmission der Infrarotstrahlung versehen sind. Beispielsweise sind zwei dieser Pixel mit einem Filter versehen, welcher Infrarotstrahlung nur im Wellenlängenbereich der Absorptionsbande von Kohlenstoffdioxid durchlässt. Diese Pixel dienen dann zur Detektion von Kohlenstoffdioxid in der Absorptionsstrecke. Eine alternative Möglichkeit ist, die spezifischen Absorptionsschichten direkt auf die Einzelpixel aufzubringen. Diese Absorptionsschichten können auf eine bestimmte Wellenlänge optimiert werden, so dass der Effekt vom optischen Filter entsteht.

Gemäß einer weiteren Ausführungsform der vorliegenden Erfindung sind zumindest zwei Pixel des Sensorarrays ausgebildet sind, ein zweites Gas zu detektieren, und mit einem zweiten optischen Filter zur wellenlängenselektiven Transmission der Infrarotstrahlung versehen. Beispielsweise weist das Sensorarray vier Zeilen Pixel, und vier Spalten Pixel auf, wobei neun der sechzehn Pixel mit dem zweiten Filter zur wellenlängenselektiven Transmission der Infrarotstrahlung versehen sind. Beispielsweise sind neun dieser Pixel mit einem Filter versehen, welcher Infrarotstrahlung nur im Wellenlängenbereich der Absorptionsbande von Wasser durchlässt. Diese Pixel dienen dann zur Detektion von Wasser in der Absorptionsstrecke. Die Auswerteeinrichtung kann dann einen zweiten Messkanal aufweisen, welcher die zusammengefassten Pixelmessignale für das zweite Gas ausgibt.

Gemäß einer weiteren Ausführungsform der vorliegenden Erfindung ist zumindest ein Pixel des Sensorarrays als Überwachungs- und Abgleich-Pixel ausgebildet. Beispielsweise weist das Sensorarray acht Zeilen Pixel, und acht Spalten Pixel auf, wobei neun der vierundsechzig Pixel mit dem ersten Filter, weitere neun Pixel mit dem zweiten Filter, weitere neun der Pixel mit dem Referenz-Filter versehen sind, und die verbleibenden Pixel mit keinem Filter versehen sind. Das Überwachungs- und Abgleich-Pixel ist ausgebildet, um z.B. die Leistung der Infrarot-Strahlungsquelle und/oder den Verschmutzungsgrad der Messtrecke zu überwachen. Auch kann eine Alterung der Infrarot-Strahlungsquelle und/oder eine Verschiebung des abgestrahlten Wellenlängenbereichs erfasst und gegebenenfalls mittels der Auswerteeinrichtung korrigiert werden. Dies kann gemäß der Erfindung selektiv für jedes einzelne Pixel des Sensorarrays erfolgen.

Gemäß einer weiteren Ausführungsform der vorliegenden Erfindung ist die Auswerteeinrichtung ausgebildet, Pixelmesssignale von Pixeln in einem oder mehreren Pixelbereichen anhand mindestens eines vorgegebenen Kriteriums beim Zusammenfassen der Pixelmessignale nicht zu berücksichtigen. So läßt sich die Messgenauigkeit erhöhen.

Gemäß einer weiteren Ausführungsform der vorliegenden Erfindung besteht das vorbestimmte Kriterium darin, dass eine Abweichung des Pixelmesssignals von dem Mittelwert der Pixelmesssignale größer als ein vorbestimmter Betrag ist. Beispielsweise werden Pixelmesssignale von Pixeln nicht berücksichtigt, welche eine Abweichung von mehr als 5%, 10%, 20%, 30%, 40% oder 50% vom Mittelwert der Pixelmesssignale aufweisen. Auch können z. B. nur Pixelmesssignale berücksichtigt werden, welcher innerhalb einer Standardabweichung σ, zwei Standardabweichungen 2σ, oder drei Standardabweichungen 3σ vom Mittelwert liegen. Auch Einflussfaktoren, wie z.B. die Empfindlichkeit der einzelnen Pixel, und/oder der Offset der Pixelmesssignale der einzelnen Pixel, und/oder die Verstärkung der einzelnen Pixel, können bei der Zusammenfassung der Pixelmesssignale für den einzelnen Messkanal berücksichtig werden. Falls diese Komponenten der Pixelmesssignale von einzelnen Pixeln zu deutlich abweichen, werden diese einzelnen Pixel bei einer Zusammenfassung der Pixelmesssignale nicht berücksichtigt.

Gemäß einer weiteren Ausführungsform der vorliegenden Erfindung ist die Auswerteeinrichtung mit dem Sensorarray einstückig ausgebildet. Beispielsweise sind die Auswerteinrichtung und das Sensorarray als ASIC (application-specific integrated circuit) ausgebildet. Die Auswerteinrichtung kann jedoch auch als externer Mikrocontroller ausgebildet sein, und mit den einzelnen Pixeln jeweils über eine Sensorleitung gekoppelt sein. Das Sensorarray kann beispielsweise aus einem CCD (Charge-Coupled Device) -Sensorarray oder in CMOS-Technik ausgebildet sein. Die Pixel können auch jeweils eine eigene Auswerteschaltung aufweisen, welche zusammengeschaltet die Auswerteinrichtung bilden.

Gemäß einer weiteren Ausführungsform der vorliegenden Erfindung ist die Auswerteeinrichtung ausgebildet, die Verstärkung und/oder den Offset der einzelnen Pixel zu verändern. Da die Pixel des Sensorarrays einzeln ansteuerbar ausgebildet sind, kann auf diese Weise auf einfache Art die Messgenauigkeit verbessert werden.

Die beschriebenen Ausgestaltungen und Weiterbildungen lassen sich beliebig miteinander kombinieren.

### Kurze Beschreibung der Zeichnungen

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung.

Andere Ausführungsformen und viele der genannten Vorteile ergeben sich im Hinblick auf die Zeichnungen. Die dargestellten Elemente der Zeichnungen sind nicht notwendigerweise maßstabsgetreu zueinander gezeigt.

Es zeigen:
- Fig. 1: ein schematisches Schnittbild einer spektroskopischen Sensorvorrichtung gemäß einer Ausführungsform der vorliegenden Erfindung;
- Fig. 2: ein schematisches Schnittbild einer spektroskopischen Sensorvorrichtung gemäß einer weiteren Ausführungsform der vorliegenden Erfindung;
- Fig. 3: eine schematische Draufsicht eines Infrarot-Sensorarrays einer spektroskopischen Sensorvorrichtung gemäß einer weiteren Ausführungsform der vorliegenden Erfindung;
- Fig. 4: eine schematische Draufsicht eines Infrarot-Sensorarrays einer spektroskopischen Sensorvorrichtung gemäß einer weiteren Ausführungsform der vorliegenden Erfindung;
- Fig. 5: eine schematische Draufsicht eines Infrarot-Sensorarrays einer spektroskopischen Sensorvorrichtung gemäß einer weiteren Ausführungsform der vorliegenden Erfindung; und
- Fig. 6: ein schematisches Flussdiagramm eines Verfahrens zum Betreiben einer spektroskopischen Sensorvorrichtung gemäß einer weiteren Ausführungsform der vorliegenden Erfindung.

In den Figuren der Zeichnungen bezeichnen gleiche Bezugszeichen gleiche oder funktionsgleiche Elemente, Bauteile oder Komponenten, soweit nichts Gegenteiliges angegeben ist.

### Ausführungsformen der Erfindung

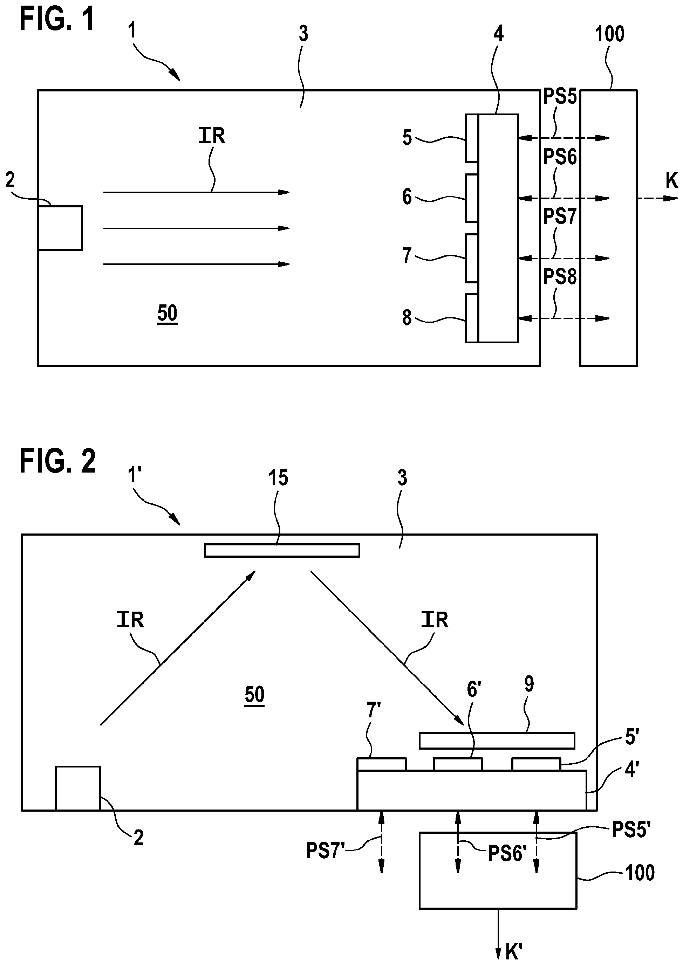

Fig. 1 zeigt ein schematisches Schnittbild einer spektroskopischen Sensorvorrichtung 1 gemäß einer Ausführungsform der vorliegenden Erfindung.

Die spektroskopische Sensorvorrichtung 1 weist eine Absorptionsstrecke 3 auf, in welcher sich zumindest ein zu detektierendes Fluid 50, z. B. gasförmiges Kohlenstoffdioxid, befindet. Die einer spektroskopischen Sensorvorrichtung 1 weist eine Infrarot-Strahlungsquelle 2 auf, welche ausgebildet ist, Infrarotstrahlung IR in die Absorptionsstrecke 3 zu strahlen. Die Infrarot-Strahlungsquelle 2 kann eine breitbandige Strahlungsquelle, wie z. B. eine Leuchtdiode oder eine Glühbirne, sein, und elektromagnetische Strahlung in einem Wellenlängenbereich von etwa 700 Nanometer bis 2 Millimeter, vorzugsweise zwischen 780 Nanometer bis 1 Millimeter aussenden. Die Infrarot-Strahlungsquelle 2 kann jedoch auch als monochromatische Strahlungsquelle ausgebildet sein, welche lediglich elektromagnetische Strahlung im Absorptionsbereich des zu untersuchendes Fluids 50 ausstrahlt.

Des Weiteren weist die spektroskopischen Sensorvorrichtung 1 ein Infrarot-Sensorarray 4 auf, welche eine Matrix mit einer Vielzahl von einzeln auswertbaren und ansteuerbaren Pixeln 5, 6, 7, 8 aufweist, welche ausgebildet sind, die von der Infrarot-Strahlungsquelle 2 ausgesendete und durch das Fluid propagierte Infrarotstrahlung IR als einzelne Pixelmessignale zu detektieren.

Überdies weist die spektroskopischen Sensorvorrichtung 1 eine Auswerteeinrichtung 100 auf, welche ausgebildet ist, eine Mehrzahl von einzelnen Pixelmessignalen der Pixeln 5, 6, 7, 8 zusammenzufassen, und über einen einzelnen Messkanal K auszugeben. Die in der Fig. 1 dargestellte spektroskopischen Sensorvorrichtung 1 weist vier Pixel 5, 6, 7, 8 auf, welche jeweils einzeln auswertbar und ansteuerbar ausgebildet sind und die über Sensorleitungen PS5, PS6, PS7 und PS8 mit der Auswerteeinrichtung 100 gekoppelt sind.

Die Auswerteeinrichtung 100 ist gemäß dieser Ausführungsform der Erfindung ausgebildet, Pixelmesssignale von einzelnen Pixeln, welche eine Abweichung größer als einen vorherbestimmten Betrag von dem Mittelwert der Pixelmesssignale aufweisen, beim Zusammenfassen der Pixelmesssignale nicht zu berücksichtigen. Dies bedeutet, dass falls beispielsweise das Pixel 5 und das Pixel 8 des Infrarot-Sensorarrays 4 ein fehlerhaftes Pixelmessignal über die Sensorleitungen PS5 und PS8 an die Auswerteeinrichtung 100 liefern, die Pixel 5 und 8 beim Zusammenfassen der Pixelmesssignale des Infrarot-Sensorarrays 4 nicht berücksichtigt werden oder von der Auswerteeinrichtung 100 über die Sensorleitungen PS5 und PS8 ausgeschaltet werden.

Auch können die einzelnen Pixel 5, 6, 7, 8 mittels der Auswerteeinrichtung 100 derart angesteuert werden, sodass sich z.B. die Pixelmessignale der Pixel 5 und 8 korrigieren lassen. Falls beispielsweise die Pixel 5 und 8 einen Offset-Fehler aufweisen, kann der Offset-Fehler der Pixel 5 und 8 separat korrigiert werden. Auch kann beispielsweise die Empfindlichkeit und/oder die Verstärkung der Pixel 5 und 8 fehlerhaft sein, und bei bedarf mittels der Auswerteinrichtung 100 korrigiert werden.

Die Auswerteeinrichtung 100 ist in der Fig. 1 getrennt von dem Infrarot-Sensorarray 4, z.B. als externer Mikrocontroller, ausgebildet. Jedoch kann die Auswerteeinrichtung 100 auch einstückig mit dem Infrarot-Sensorarray 4 ausgebildet sein oder in den Pixel integriert sein.

Bei dieser Ausführungsform ist die Korrekturfunktion in der Auswerteeinrichtung 100 vorprogrammiert, z.B. bei der Endkontrolle. In einer weiteren Ausgestaltung ist es möglich, dass die Korrekturfunktion durch eine (nicht dargestellte) externe Eingabeeinrichtung nachträglich änderbar ist bzw. eine Selbstadaptionsfunktion aufweist.

Fig. 2 zeigt ein schematisches Schnittbild einer spektroskopischen Sensorvorrichtung 1' gemäß einer weiteren Ausführungsform der vorliegenden Erfindung. In dieser Ausführungsform wird die Infrarotstrahlung IR der Infrarot-Strahlungsquelle 2 über einen Reflektor 15 hin zu dem Infrarot-Sensorarray 4' gelenkt. Auf diese Weise kann die Absorptionsstrecke 3 verlängert werden. Die Pixel 5' und 6' des Infrarot-Sensorarrays 4 liefern Pixelmesssignale an die Auswerteeinrichtung 100, welche die zusammengefassten Pixelmesssignale über den einzigen Messkanal K' ausgibt.

In der dargestellten Ausführungsform ist über den Pixeln 5' und 6' ein optischer Filter 9 angeordnet, welcher zur wellenlängenselektiven Transmission der Infrarotstrahlung ausgebildet ist. Beispielsweise ist der Filter 9 ausgebildet, nur Wellenlängen durchzulassen, welche für eine Messung von Kohlenstoffdioxid relevant sind. Je nach Anwendung und zu detektierenden Gas kann der Filter 9 und das Transmissionsspektrum geeignet ausgewählt werden. Der Filter 9 kann beispielsweise durch Aufkleben mit dem Infrarot-Sensorarray 4' und den entsprechenden Pixeln 5', 6' verbunden sein.

Das Pixel 7' des Infrarot-Sensorarrays 4', welches nicht mit dem Filter 9 bedeckt ist, kann beispielsweise als Referenzpixel zum Abgleich, zur Kalibrierung bzw. zur Überwachung der einer spektroskopischen Sensorvorrichtung 1' verwendet werden. Zum Beispiel kann mit dem Pixel 7' die Leistung der Infrarot-Strahlungsquelle 2 oder der Verschmutzungsgrad des Reflektors 15 ermittelt werden.

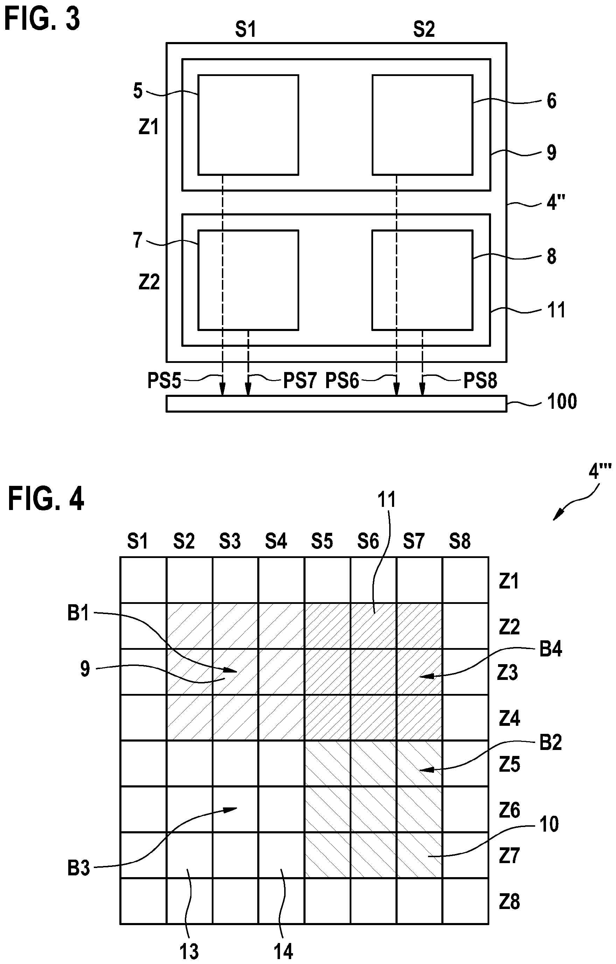

Fig. 3 zeigt eine beispielhafte Draufsicht eines Infrarot-Sensorarrays 4" einer spektroskopischen Sensorvorrichtung gemäß einer weiteren Ausführungsform der vorliegenden Erfindung.

In dieser Ausführungsform weist das Infrarot-Sensorarray 4" zwei Zeilen Pixel Z1 und Z2, und zwei Spalten Pixel S1 und S2 auf. Das Infrarot-Sensorarray 4" weist auf diese Weise vier quadratisch angeordnete Pixel 5, 6, 7 und 8 auf. Die Pixel 5 und 6 sind mit einem optischen Filter 9 versehen, welcher lediglich Strahlung durchlässt, welche in der Absorptionsbande von Kohlenstoffdioxid liegt. Die Pixel 7 und 8 sind mit einem optischen Referenzfilter 11 versehen. Da jedes Pixel 5, 6, 7 und 8 einzeln mittels der Auswerteeinrichtung 100 ansteuerbar ist, kann mittels der Auswerteinrichtung 100 jederzeit geprüft werden, ob die beiden Pixel 5 und 6 für die Gasmessung, und/oder die beide Pixel 7 und 8 für den Abgleich unterschiedliche Pixelmesssignale aufweisen. Weisen die Pixel 5 und 6 oder die Pixel 7 und 8 ein unterschiedliches Signal auf, so liegt ein Fehler vor.

Dieser Fehler kann beispielsweise seine Ursache in einer lokalen Verschmutzung durch ein Partikel auf dem Filter 9 oder 11, und/oder in einer fehlerhaften Sensorleitung PS5, PS6, PS7, PS8 und/oder in dem Versagen der Auswerteschaltung für das jeweilige Pixel 5, 6, 7 und 8 haben. Ob ein Fehler vorliegt, kann so mittels der Auswerteinrichtung 100 überprüft werden.

Durch die höhere Anzahl an Pixel kann die Empfindlichkeit der Messung für das Gas durch die Pixel 5 und 6, und für den Referenzwert durch die Pixel 7 und 8 additiv erhöht werden. Ferner kann die Messung auf diese Weise deutlich verfeinert werden. Des Weiteren kann durch diese Ausbildung eines ein defekter Pixel sicher erkannt und gegeben falls mittels der Auswerteinrichtung 100 abgeschaltet oder korrigiert werden.

Fig. 4 zeigt eine beispielhafte Draufsicht eines Infrarot-Sensorarrays 4'" einer spektroskopischen Sensorvorrichtung gemäß einer weiteren Ausführungsform der vorliegenden Erfindung. Das Infrarot-Sensorarray 4'" weist in dieser Ausführungsform acht Zeilen Z1- Z8 einzeln auswertbarer Pixel, und acht Spalten S1 - S8 einzeln auswertbarer Pixel auf. In dieser Ausführungsform ist das Infrarot-Sensorarray 4" ausgebildet, gleichzeitig zwei unterschiedliche Gase in der Absorptionsstrecke 3 zu analysieren.

Beispielsweise ist ein erster Pixelbereich B1, welcher die Zeilen Z2 -Z4, und die Spalten S2 - S4 umfasst, mit einem ersten optischen Filter 9 bedeckt. Der optische Filter 9 kann ausgebildet sein, um für Wellenlängen in der Absorptionsbande von Kohlenstoffdioxid transparent zu sein. Der Pixelbereich B1 umfasst somit neun Pixel, die einzeln ausgewertet werden und über einen ersten Messkanal ausgegeben werden können.

Da diese Pixel im Pixelbereich B1 mit dem gleichen Filter 9 versehen sind, muss bei diesen neun Pixeln nach der Herstellung der spektroskopischen Sensorvorrichtung 1 bei einer Überprüfung ein gleiches Pixellmesssignal anliegen. Wird bei einem Pixel eine deutliche Abweichung durch Vergleich der Pixelmesssignale der neun Pixel in der Auswerteeinrichtung 100 festgestellt, so muss bei diesem Pixel ein Defekt vorliegen. Dieser Pixel kann dann mittels der Auswerteeinrichtung 100 bei der Messung ausgeschlossen werden, wobei die Funktionalität danach ohne Fehler weiter erfüllt werden kann.

In der Fig. 4 ist zu erkennen, dass das Infrarot-Sensorarray 4'" mit unterschiedlichen optischen Filtern 9, 10 ,11 versehen ist. Ferner sind Pixel des Infrarot-Sensorarrays 4 nicht mit einem Filter versehen. Die Pixelbereiche ohne Filter können für bestimmte Zusatzfunktionen, z.B. als Referenzpixel, verwendet werden.

Der Pixelbereich B2 ist mit einem optischen Filter 10 versehen, dessen Transmissionsbereich im Bereich der Wasser-Absorptionsbande liegt. Auch der Pixelbereich B2 umfasst neun einzeln auswertbare Pixel, und erstreckt zwischen den Zeilen Z5 - Z7, und den Spalten S5 - S7. Mit diesen Pixeln kann die z. B. der Wasseranteil eines Gases detektiert werden und über einen zweiten Messkanal ausgegeben werden.

Die Pixel, z. B. das Pixel 13 und das Pixel 14 im dritten Pixelbereich B3, welche nicht mit einem Filter versehen sind, können beispielsweise dazu verwendet werden, um die Leistung der Infrarot-Strahlungsquelle 2 über die Lebensdauer der spektrokopischen Sensorvorrichtung zu überwachen und bei Bedarf mittels der Auswerteinrichtung 100 zu korrigieren.

Der vierte Pixelbereich B4, welcher zwischen den Zeilen Z2 - Z4, und den Spalten S5 - S7 angeordnet ist, ist mit einem optischen Referenz-Filter 12 versehen, und dient zur Überwachung der Pixelmesssignale, welche von den Pixelbereichen B1 und B2 erhalten werden. Auch im Pixelbereich B4 sind neun Pixel angeordnet, welche einzeln auswertebar und ansteuerbar sind.

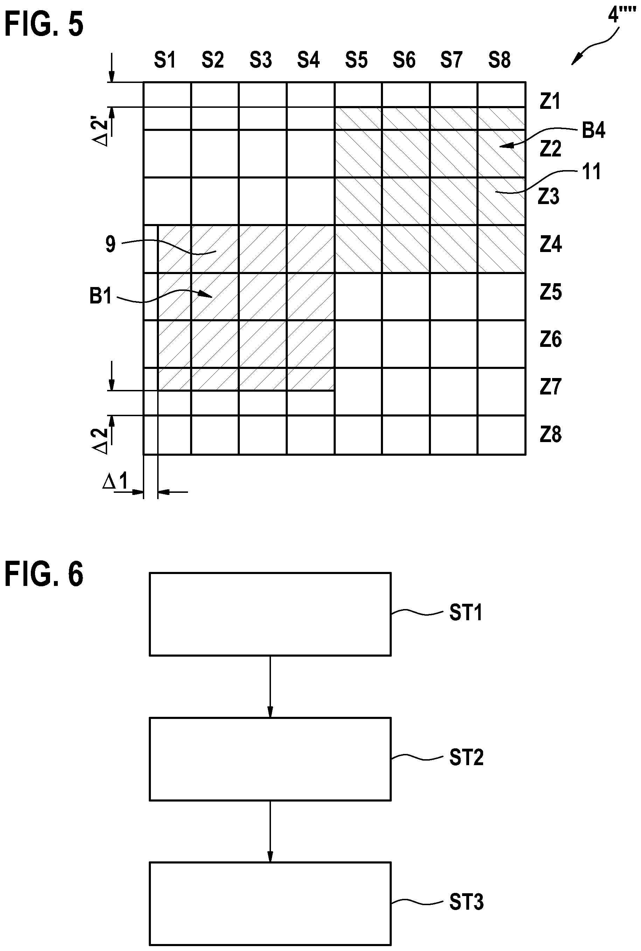

Fig. 5 zeigt eine beispielhafte Draufsicht eines Infrarot-Sensorarrays 4'" einer spektroskopischen Sensorvorrichtung gemäß einer weiteren Ausführungsform der vorliegenden Erfindung.

Bei dieser Ausführungsform kann die Auswerteeinrichtung 100 die genaue Position des ersten optischen Filters 9, und/oder des Referenzfilters 11 auf den Pixeln des Infrarot-Sensorarrays 4 bestimmten kann, und dazu beitragen, dass die Anforderungen an die Fertigungstoleranzen deutlich minimiert werden können, indem fehlerhaft bedeckte Pixel von der Auswertung eliminiert werden.

Wird, wie in Fig. 5 schematisch dargestellt, ein optischer Filter 9 verwendet, dessen Dimension so gewählt ist, dass es sichergestellt ist, dass zumindest vier Pixel bedeckt werden, jedoch die Position des Filters 9 über den Pixeln fertigungsbedingt nicht exakt stimmt, so kann beim Auswerten der Pixelmesssignale der einzelnen Pixel die Position des Filters durch einen Vergleich der Pixelmesssignale der einzelnen Pixel bestimmt werden, und in der Auswerteeinrichtung 100 in einem Speicher abgespeichert werden.

Der Filter 9 des Infrarot-Sensorarrays 4"", welcher beispielsweise transparent für Wellenlängen in der Absorptionsbande von Methan ist, ist so dimensioniert, dass er neun Pixel vollständig abdecken kann. Kommt es nun bei der Fertigung beim Aufbringen des optischen Filters 9 auf das Infrarot-Sensorarrays 4"" zu einem Fehler bei der Positionierung des Filters 9 auf den Pixeln, so kann mittels der Auswerteinrichtung 100 überprüft werden, welche der Pixel im Bereich zwischen den Zeilen Z4 - Z8 und den Spalten S1- S4 einen innerhalb einer vorgegeben Toleranz befindlichen Pixelmesssignal aufweisen. Die Auswerteinrichtung 100 erkennt beispielsweise, dass die Pixel im Bereich zwischen den Zeilen Z4 - Z6 und im Bereich zwischen den Spalten S2 - S4 jeweils Pixelmesssignale ausgeben, welche innerhalb einer Toleranz von 10% vom Mittelwert liegen.

Die Auswerteeinrichtung 100 erkennt dies, und berücksichtigt bei einer Messung eines Gases lediglich die Pixelmesssignale, welche von Pixeln ausgegeben wurden, welche sich im Bereich zwischen den Zeilen Z4 - Z6 und im Bereich zwischen den Spalten S2 - S4 befinden.

Da die Pixel zwischen den Zeilen Z4 - Z7 in Spalte S1 nur teilweise von dem Filter 9 verdeckt sind, da der Filter horizontal um Δ1 versetzt angeordnet ist, und die Pixel zwischen den Spalten S1 und S4 in Zeile Z7 ebenfalls nur teilweise von dem Filter 9 verdeckt sind, da der Filter vertikal um Δ2 versetzt angeordnet ist, geben diese Pixel ein Pixelmesssignal an die Auswerteeinrichtung 100 aus, welcher außerhalb der Toleranzgrenze ist. Die Pixel zwischen den Zeilen Z4 - Z7 in Spalte S1 und zwischen den Spalten S1 und S4 in Zeile Z7 werden daher bei einer Messung nicht berücksichtigt.

In analoger Weise kann die genaue Position des Referenzfilters 11 im Pixelbereich B4 bestimmt werden. Auch der Referenzfilter 11 ist in vertikaler Richtung um Δ2' versetzt angeordnet. Daher werden die Pixel zwischen Spalte S5 - S8 in Zeile Z1 nur teilweise von dem Referenzfilter 11 abgedeckt. Die Auswerteeinrichtung 100 kann daher anhand der von den Pixeln im Pixelbereich B4 erhalten Pixelmesssignale die genaue Position des Referenzfilters 11 auf den Pixeln bestimmen, und nur die Pixel im Bereich in zwischen Zeile Z2 -Z4 und Spalten S3 -S8 berücksichtigen.

Fig. 6 zeigt ein schematisches Flussdiagramm eines Verfahrens zum Betreiben einer spektroskopischen Sensorvorrichtung gemäß einer weiteren Ausführungsform der vorliegenden Erfindung.

In einem ersten Verfahrensschritt ST1 erfolgt ein Messen der Pixelmesssignale der Pixel in einem oder mehreren Pixelbereichen mit jeweils einer Mehrzahl von Pixeln eines Infrarot-Sensorarrays.

In einem zweiten Verfahrensschritt ST2 erfolgt ein Analysieren der Pixelmesssignale anhand mindestens eines vorgegebenen Kriteriums. Beispielsweise erfolgt ein Berechnen des Mittelwerts der Pixelmesssignale und ein Ermitteln einer jeweiligen Abweichung der einzelnen Pixelmesssignale davon.

In einem dritten Verfahrensschritt ST3 erfolgt ein Zusammenfassen der Pixelmesssignale der einzelnen Pixel in einem jeweiligen Bereich unter Berücksichtigung des Ergebnisses des Analysierens. Beispielsweise werden Pixelmesssignale von einzelnen Pixeln, welche eine Abweichung größer als ein vorherbestimmter Betrag von dem Mittelwert der Pixelmessignale aufweisen, beim Zusammenfassen nicht berücksichtigt.

Obwohl die vorliegende Erfindung anhand bevorzugter Ausführungsbeispiele vorstehend beschrieben wurde, ist sie nicht darauf beschränkt, sondern auf vielfältige Art und Weise modifizierbar. Insbesondere lässt sich die Erfindung in mannigfaltiger Weise verändern oder modifizieren, ohne vom Kern der Erfindung abzuweichen.

## Patentansprüche

1. Spektroskopische Sensorvorrichtung (1; 1') mit:
einer Absorptionsstrecke (3) zum Aufnehmen zumindest eines zu analysierenden fluiden Mediums,
einer Infrarot-Strahlungsquelle (2) zum Aussenden von Infrarotstrahlung in die Absorptionsstrecke (3),
einem Infrarot-Sensorarray (4; 4'; 4"; 4"'; 4""), welches eine Vielzahl von einzeln auswertbaren Pixeln (5, 6, 7, 8, 9; 5', 6' ,7') aufweist, welche ausgebildet sind, die von der Infrarot-Strahlungsquelle (2) durch das fluide Medium propagierte Infrarotstrahlung als einzelne Pixelmesssignale zu detektieren, wobei die Pixel ein oder mehrerer Pixelbereiche (B1, B2, B4; B1, B4) zumindest teilweise mit einem jeweiligen optischen Filter (9, 10, 11; 9, 11) zur wellenlängenselektiven Transmission der Infrarotstrahlung versehen sind; und
einer Auswerteeinrichtung (100), welche ausgebildet ist, eine Mehrzahl von Pixelmessignalen der Pixel (5, 6) zusammenzufassen, und über einen einzelnen Messkanal (K; K') auszugeben, wobei die Auswerteeinrichtung (100) ausgebildet ist, die Position des oder der optischen Filter (9, 10, 11; 9, 11) zu bestimmen.

2. Spektroskopische Sensorvorrichtung (1; 1') nach Anspruch 1,
wobei zumindest zwei Pixel (5, 6,) des Infrarot-Sensorarrays (4; 4'; 4"; 4"'; 4"") ausgebildet sind, ein erstes Gas zu detektieren, und mit einem ersten optischen Filter (9) zur wellenlängenselektiven Transmission der Infrarotstrahlung versehen sind.

3. Spektroskopische Sensorvorrichtung (1; 1') nach Anspruch 2,
wobei zumindest zwei Pixel (7, 8) des Infrarot-Sensorarrays (4; 4'; 4"; 4"'; 4"") ausgebildet sind, ein zweites Gas zu detektieren, und mit einem zweiten optischen Filter (10) zur wellenlängenselektiven Transmission der Infrarotstrahlung versehen sind.

4. Spektroskopische Sensorvorrichtung (1; 1') nach einem der vorhergehenden Ansprüche, wobei zumindest ein Pixel des Infrarot-Sensorarrays (4; 4'; 4"; 4"'; 4"") als Überwachungs- und Abgleich-Pixel ausgebildet ist.

5. Spektroskopische Sensorvorrichtung (1; 1') nach einem vorhergehenden Ansprüche, wobei die Auswerteeinrichtung (100) ausgebildet ist, Pixelmesssignale von Pixeln in einem oder mehreren Pixelbereichen (B1, B2, B3, B4) anhand mindestens eines vorgegebenen Kriteriums beim Zusammenfassen der Pixelmesssignale nicht zu berücksichtigen.

6. Spektroskopische Sensorvorrichtung (1; 1') nach Anspruch 5, wobei das vorbestimmte Kriterium darin besteht, dass eine Abweichung des Pixelmesssignals von dem Mittelwert der Pixelmesssignale größer als ein vorherbestimmter Betrag ist.

7. Spektroskopische Sensorvorrichtung (1; 1') nach einem der vorhergehenden Ansprüche, wobei die Auswerteeinrichtung (100) mit dem Infrarot-Sensorarray (4; 4'; 4"; 4"'; 4"") einstückig ausgebildet ist.

8. Spektroskopische Sensorvorrichtung nach einem der vorhergehenden Ansprüche, wobei die Auswerteeinrichtung (100) ausgebildet ist, die Verstärkung oder den Offset der einzelnen Pixel (5, 6, 7, 8, 9; 5', 6' ,7') zu verändern.

9. Verfahren zum Betreiben einer spektroskopischen Sensorvorrichtung (1; 1') nach Anspruch 1 mit den Schritten:
Messen (ST1) der Pixelmesssignale der Pixel in einem oder mehreren Pixelbereichen (B1, B2, B3, B4) mit jeweils einer Mehrzahl von Pixeln des Infrarot-Sensorarrays (4; 4'; 4"; 4"'; 4""),
Analysieren (ST2) der Pixelmesssignale anhand mindestens eines vorgegebenen Kriteriums,
Bestimmen der Position des oder der optischen Filter (9, 10, 11; 9, 11) mittels der Auswerteeinrichtung, und
Zusammenfassen (ST3) der Pixelmesssignale der einzelnen Pixel in einem jeweiligen Bereich unter Berücksichtigung des Ergebnisses des Analysierens.

## Claims

1. Spectroscopic sensor apparatus (1; 1'), having:
absorption section (3) for holding at least one fluid medium to be analysed,
an infrared radiation source (2) for emitting infrared radiation into the absorption section (3),
an infrared sensor array (4; 4'; 4"; 4"'; 4""), having a multiplicity of individually evaluable pixels (5, 6, 7, 8, 9; 5;', 6', 7') that are configured to detect the infrared radiation, propagating from the infrared radiation source (2) through the fluid medium, as individual pixel measurement signals, wherein the pixels of one or more pixel regions (B1, B2, B4; B1, B4) are at least partially provided with a respective optical filter (9, 10, 11; 9, 11) for the wavelength-selective transmission of the infrared radiation; and
an evaluation device (100), configured to combine a plurality of pixel measurement signals of the pixels (5, 6) and to output them via an individual measurement channel (K; K'), wherein the evaluation device (100) is configured to determine the position of the optical filter or filters (9, 10, 11; 9, 11).

2. Spectroscopic sensor apparatus (1; 1') according to Claim 1, wherein at least two pixels (5, 6) of the infrared sensor array (4; 4' ; 4"; 4"'; 4"") are configured to detect a first gas and are provided with a first optical filter (9) for the wavelength-selective transmission of the infrared radiation.

3. Spectroscopic sensor apparatus (1; 1') according to Claim 2, wherein at least two pixels (7, 8) of the infrared sensor array (4; 4'; 4"; 4"'; 4"") are configured to detect a second gas and are provided with a second optical filter (10) for the wavelength-selective transmission of the infrared radiation.

4. Spectroscopic sensor apparatus (1; 1') according to one of the preceding claims, wherein at least one pixel of the infrared sensor array (4; 4'; 4"; 4"'; 4"") is configured as a monitoring and adjustment pixel.

5. Spectroscopic sensor apparatus (1; 1') according to one of the preceding claims, wherein the evaluation device (100) is configured to not take into account pixel measurement signals of pixels in one or more pixel regions (B1, B2, B3, B4) based on at least one specified criterion when combining the pixel measurement signals.

6. Spectroscopic sensor apparatus (1; 1') according to Claim 5, wherein the predetermined criterion is that a deviation of the pixel measurement signal from the average value of the pixel measurement signals is greater than a previously determined absolute value.

7. Spectroscopic sensor apparatus (1; 1') according to one of the preceding claims, wherein the evaluation device (100) is formed in one piece with the infrared sensor array (4; 4'; 4"; 4"'; 4"").

8. Spectroscopic sensor apparatus according to one of the preceding claims, wherein the evaluation device (100) is configured to change the amplification or the offset of the individual pixels (5, 6, 7, 8, 9; 5', 6', 7').

9. Method for operating a spectroscopic sensor apparatus (1; 1') according to Claim 1, having the steps of:
measuring (ST1) the pixel measurement signals of the pixels in one or more pixel regions (B1, B2, B3, B4) with in each case a plurality of pixels of the infrared sensor array (4; 4'; 4"; 4"'; 4""),
analysing (ST2) the pixel measurement signals on the basis of at least one specified criterion,
determining the position of the optical filter or filters (9, 10, 11; 9, 11) using the evaluation device, and combining (ST3) the pixel measurement signals of the individual pixels in a respective region by taking into account the result of the analysis.

## Revendications

1. Arrangement détecteur spectroscopique (1 ; 1'), comprenant
un trajet d'absorption (3) destiné à accueillir au moins un milieu fluide à analyser,
une source de rayonnement infrarouge (2) destinée à émettre un rayonnement infrarouge dans le trajet d'absorption (3),
un réseau de capteurs d'infrarouge (4 ; 4' ; 4" ; 4"' ; 4""), qui possède une pluralité de pixels (5, 6, 7, 8, 9 ; 5', 6', 7') pouvant être interprétés individuellement, lesquels sont configurés pour détecter le rayonnement infrarouge qui se propage à travers le milieu fluide depuis la source de rayonnement infrarouge (2) en tant que signaux de mesure de pixel individuels, les pixels d'une ou plusieurs zones de pixels (B1, B2, B4 ; B1, B4) étant au moins partiellement pourvus d'un filtre optique (9, 10, 11 ; 9, 11) respectif servant à la transmission sélective en longueur d'onde du rayonnement infrarouge ; et
un dispositif d'interprétation (100), qui est configuré pour regrouper une pluralité de signaux de mesure de pixel des pixels (5, 6) et les délivrer en sortie par le biais d'un canal de mesure (K ; K') unique, le dispositif d'interprétation (100) étant configuré pour déterminer la position du ou des filtres optiques (9, 10, 11 ; 9, 11) .

2. Arrangement détecteur spectroscopique (1 ; 1') selon la revendication 1, au moins deux pixels (5, 6) du réseau de capteurs d'infrarouge (4 ; 4' ; 4" ; 4"' ; 4"") étant configurés pour détecter un premier gaz et étant pourvus d'un premier filtre optique (9) servant à la transmission sélective en longueur d'onde du rayonnement infrarouge.

3. Arrangement détecteur spectroscopique (1 ; 1') selon la revendication 2, au moins deux pixels (7, 8) du réseau de capteurs d'infrarouge (4 ; 4' ; 4" ; 4"' ; 4"") étant configurés pour détecter un deuxième gaz et étant pourvus d'un deuxième filtre optique (10) servant à la transmission sélective en longueur d'onde du rayonnement infrarouge.

4. Arrangement détecteur spectroscopique (1 ; 1') selon l'une des revendications précédentes, au moins un pixel du réseau de capteurs d'infrarouge (4 ; 4' ; 4" ; 4"' ; 4"") étant configuré sous la forme d'un pixel de surveillance et de compensation.

5. Arrangement détecteur spectroscopique (1 ; 1') selon l'une des revendications précédentes, le dispositif d'interprétation (100) étant configuré pour ne pas tenir compte des signaux de mesure de pixel des pixels dans une ou plusieurs zones de pixels (B1, B2, B3, B4) à l'aide d'au moins un critère prédéfini lors du regroupement des signaux de mesure de pixel.

6. Arrangement détecteur spectroscopique (1 ; 1') selon la revendication 5, le critère prédéfini consistant en ce qu'un écart entre le signal de mesure de pixel et la valeur moyenne des signaux de mesure de pixel est supérieur à une valeur absolue préalablement déterminée.

7. Arrangement détecteur spectroscopique (1 ; 1') selon l'une des revendications précédentes, le dispositif d'interprétation (100) étant réalisé d'un seul tenant avec le réseau de capteurs d'infrarouge (4 ; 4' ; 4" ; 4"' ; 4"").

8. Arrangement détecteur spectroscopique selon l'une des revendications précédentes, le dispositif d'interprétation (100) étant configuré pour modifier l'amplification ou le décalage des pixels (5, 6, 7, 8, 9 ; 5', 6', 7') individuels.

9. Procédé pour faire fonctionner un arrangement détecteur spectroscopique (1 ; 1') selon la revendication 1, comprenant les étapes suivantes :
mesure (ST1) des signaux de mesure de pixel des pixels dans une ou plusieurs zones de pixels (B1, B2, B3, B4) respectivement avec une pluralité de pixels du réseau de capteurs d'infrarouge (4 ; 4' ; 4" ; 4"' ; 4""),
analyse (ST2) des signaux de mesure de pixel à l'aide d'au moins un critère prédéfini,
détermination de la position du ou des filtres optiques (9, 10, 11 ; 9, 11) au moyen du dispositif d'interprétation, et
regroupement (ST3) des signaux de mesure de pixel des pixels individuels dans une zone respective en tenant compte du résultat de l'analyse.
